# EUROPEAN PATENT SPECIFICATION

(11) **EP 2 007 196 B1**
(45) Date of publication and mention of the grant of the patent: **25.10.2017**
(21) Application number: 07760765.3
(22) Date of filing: 17.04.2007
(51) Int. Cl.: A61F 13/00, A61K 9/14, A01K 67/00, A61L 27/36

(54) **BONE GRAFT COMPOSITION**
KNOCHENPFROPFZUSAMMENSETZUNG
COMPOSITION DE GREFFE OSSEUSE

(30) Priority: 19.04.2006 US 407446
(43) Date of publication of application: 31.12.2008
(73) Proprietor: LIFECELL CORPORATION, Branchburg, NJ 08876 (US)
(72) Inventor: CONNOR, Jerome, Doylestown, Pennsylvania 18901 (US); QIU, Qing-Qing, Branchburg, New Jersey 08876 (US)
(74) Representative: White, Martin Paul
(86) International application number: PCT/US2007/066771
(87) International publication number: WO 2007/124302

(56) References cited:
- US-A- 5 284 655
- US-A1- 2003 143 207
- US-A1- 2006 073 592

## Description

### TECHNICAL FIELD

This invention relates to tissue engineering, and more particularly to remodeling of bone tissue.

### BACKGROUND

Bone is unique among vertebrate tissues in its ability to heal via the formation of new bone. Other tissues, e.g., muscle, heart and brain, heal by replacement with connective tissue, resulting in scar formation. Skeletal tissue regeneration is the result of a complex and dynamic interaction between three components: cells, growth factors, and a permissive scaffold. An osteoinductive scaffold has the ability to induce new bone formation by influencing the recruitment, differentiation and maturation of a patient's stem cells into bone forming cells.

US2006/073592 provides methods of storing acellular tissue matrices in which a substantial portion of water in the matrices is replaced with a water-replacing agent, e.g., glycerol. Also included in the invention of US 2006/073592 are compositions made by these methods, as well as methods of treatment using such compositions.

### SUMMARY

The present invention is directed to a method of making a bone graft composition (BGC); characterised in that said method comprises: combining fragments of an acellular tissue matrix (ATM) with fragments of demineralized bone matrix (DBM) to create a mixture, wherein the fragments of ATM and the fragments of DBM in the mixture are substantially hydrated; drying and irradiating the mixture the mixture to provide a form that has been dried and irradiated; wherein said form can be stored for six months and then rehydrated to provide an osteoinductive BCG.

The present invention is also directed to a BCG made by the method above, as well as to an article of manufacture comprising said BCG.

The inventors have found that a hydrated mixture of fragments of acellular tissue matrix (ATM) and fragments of demineralized bone matrix (DBM) can be dried to form a bone graft composition (BGC) which, when hydrated, is osteoinductive. Moreover, the inventors observed that the BGC retained osteoinductivity upon storage (e.g., long-term storage). The invention thus provides methods of making a BGC, methods of treatment using the BGC, and articles of manufacture including the BGC.

More specifically, the invention provides a method of making a bone graft composition (BGC). The method involves: (a) combining fragments (e.g., a plurality of fragments) of an acellular tissue matrix (ATM) with fragments (e.g., a plurality of fragments) of demineralized bone matrix (DBM) to create a mixture, the fragments of ATM and the fragments of DBM in the mixture being substantially hydrated; and (b) drying the mixture to form a BGC, such that, when hydrated, the BGC is osteoinductive. The fragments of ATM can be particles (e.g., particles of a uniform size) and the fragments of DBM can be particles (e.g., particles of a uniform size).

The mixture can be a semisolid putty and, prior to drying, the semisolid putty can be shaped. The ATM can be, or can include, dermis (or fascia, pericardial tissue, dura, umbilical cord tissue, placental tissue, cardiac valve tissue, ligament tissue, tendon tissue, arterial tissue, venous tissue, neural connective tissue, urinary bladder tissue, ureter tissue, or intestinal tissue) from which all, or substantially all, viable cells have been removed. The ATM can be made from human tissue or non-human mammalian (e.g., pig) tissue and the non-human mammal can be genetically engineered to lack expression of α-1,3- galactosyl residues. The genetically engineered mammal can lack a functional α-1,3-galactosyltransferase gene. Moreover, the DBM can be made from any of the above-listed mammals. The drying step can include, for example, freeze-drying and the method further involves irradiating the BGC with, e.g., γ-radiation, x-radiation, e-beam radiation, or ultraviolet radiation. The BGC can be irradiated such that it absorbs 6 kGy to 30 kGy of the radiation. Rather than, or in addition to irradiating the BGC, the mixture can be irradiated (using any of the above types and doses of radiation) prior to drying. The invention also features a BGC made by the above-described method. A method of treatment is also disclosed. The method includes: (a) identifying a mammalian subject as having a recipient organ, or tissue, in need of amelioration or repair; and (b) placing the BGC of the invention (see above) in or on the organ or tissue. The BGC can be held in place by a supportive structural device. The mixture from which the BGC was made can have been a semisolid putty and, prior to drying, the semisolid putty can have been shaped. The recipient tissue or organ can be cortical bone or cancellous bone.

The invention also provides an article of manufacture that includes: (a) the BGC of the invention (see above); and (b) packaging material, or a package insert, that includes instructions for a method of treatment. The method of treatment can involve (i) identifying a mammalian subject as having a recipient organ, or tissue, in need of amelioration or repair; and (ii) placing the BGC in or on the organ or tissue. A disclosed embodiment is a kit that includes:(a) a fragments (e.g., a plurality of fragments) of DBM; (b) fragments (e.g., a plurality of fragments) of ATM; and (c) packaging material, or a package insert, that includes instructions for a method of making a BGC. The method involves: (i) combining the fragments of ATM with the fragments of DBM to create a mixture, the fragments of ATM and the fragments of DBM in the mixture being substantially fully hydrated; and (ii) drying the mixture to form the BGC, such that, when hydrated, the BGC is osteoinductive.

Unless otherwise defined, all technical and scientific terms used herein have the same meaning as commonly understood by one of ordinary skill in the art to which this invention pertains. Preferred methods and materials are describe below, although methods and materials similar or equivalent to those described herein can be used in the practice or testing of the present invention. The materials, methods, and examples disclosed herein are illustrative only and not intended to be limiting.

Other features and advantages of the invention, *e.g*., methods of repairing bone defects, will be apparent from the following description, from the drawings and from the claims.

### DETAILED DESCRIPTION

The materials and methods provided herein can be used to make a bone graft composition (BGC) that can be implanted into damaged or defective bone tissue to facilitate the repair of the damaged or defective bone tissue. As used herein, the term "bone graft composition" is a material that a) is made from acellular tissue matrices (produced from collagen-containing tissues) and demineralized bone matrices; b) is dehydrated for long term storage; and c) retains most, and optimally all, the biological functions of the native collagen-containing and the native bone tissue from which it was made, even during long term storage.

"Bone grafting" as described herein is a procedure that places bone or bone-forming material in, on or around, or adjacent to bone defects, *e.g*., gaps, holes, or spaces in bone or breaks in bone, in order to aid in healing. Bone is a dense, multiphase material or "composite" made up of cells embedded in a matrix composed of both organic elements, including collagen fibers, lipids, peptides, glycoproteins, polysaccharides and citrates, and of inorganic elements, including calcium phosphates, carbonates, and sodium, magnesium and fluoride salts. By providing a three-dimensional scaffold, bone graft materials can act to temporarily replace missing bone and to provide a framework into, or out from, which the host bone and a vascular network can regenerate and heal. Furthermore, bone graft materials can facilitate bone repair in several ways depending upon their capacity to interact with bone forming cells.

Osteoconductive materials provide a scaffold that facilitates neovascularization and graft infiltration by way of "creeping substitution" at the edges of the graft. Osteoconductive materials are dependent on the site of implantation (*i*.*e*., they initiate new bone formation only when implanted in or on bone) and act merely as a support for new bone to bridge across the site of a defect in bone. In contrast, osteoinductive materials are distinguished by their capacity to stimulate the recipient's own pluripotent stem cells to differentiate into functioning osteogenic cells such as osteoblasts and osteoclasts. Thus, osteoinductive materials are capable of initiating new bone growth essentially independent of the implant site, *i.e.,* they can induce bone formation in any tissue, including bone, in or on which they are placed The BGC provided herein is a composition that retains osteoinductivity upon long term storage and, as such, is useful for treating a variety of bone disorders.

### I. BGC Components

Provided herein is a bone graft composition (BGC). The bone graft composition (BGC) includes an acellular tissue matrix (ATM) component and a demineralized bone matrix (DBM) component.

### Acellular tissue matrices

As used herein, an "acellular tissue matrix" ("ATM") is a tissue-derived structure that is made from any of a wide range of collagen-containing tissues by removing all, or substantially all, viable cells and, preferably, all detectable subcellular components and/or debris generated by killing cells. As used herein, an ATM lacking "substantially all viable cells" is an ATM in which the concentration of viable cells is less than 1% (*e.g*., less than: 0.1%; 0.01%; 0.001%; 0.0001%; 0.00001%; 0.000001%; or even less than 0.000001%) of that in the tissue or organ from which the ATM was made. The ATM also preferably substantially lacks dead cells and/or cellular components.

The ATM of the invention can have, or not have, an epithelial basement membrane. The epithelial basement membrane is a thin sheet of extracellular material contiguous with the basilar aspect of epithelial cells. Sheets of aggregated epithelial cells form an epithelium. Thus, for example, the epithelium of skin is called the epidermis, and the skin epithelial basement membrane lies between the epidermis and the dermis. The epithelial basement membrane is a specialized extracellular matrix that provides a barrier function and an attachment surface for epithelial-like cells; however, it does not contribute any significant structural or biomechanical role to the underlying tissue (*e.g*., dermis). Unique components of epithelial basement membranes include, for example, laminin, collagen type VII, and nidogen. The unique temporal and spatial organization of the epithelial basement membrane distinguish it from, *e.g*., the dermal extracellular matrix. The presence of the epithelial basement membrane in an ATM could be disadvantageous in that the epithelial basement membrane likely contains a variety of species-specific components that could elicit the production of antibodies, and/or bind to preformed antibodies, in xenogeneic graft recipients of the acellular matrix. In addition, the epithelial basement membrane can act as barrier to diffusion of cells and/or soluble factors (*e.g*., chemoattractants) and to cell infiltration. Its presence in an ATM can thus significantly delay formation of new tissue from the ATM in a recipient animal. As used herein, an ATM that "substantially lacks" an epithelial basement membrane is an acellular tissue matrix containing less than 5% (*e.g*., less than: 3%; 2%; 1%; 0.5%; 0.25%; 0.1%; 0.01%; 0.001%; or even less than 0.001%) of the epithelial basement membrane possessed by the corresponding unprocessed tissue from which the ATM was derived.

The ATM retain the biological and structural attributes of the tissues from which they are made, including cell recognition and cell binding as well as the ability to support cell spreading, cell proliferation, and cell differentiation. Such functions are provided by undenatured collagenous proteins (*e.g*., type I collagen) and a variety of non-collagenous molecules (*e.g*., proteins that serve as ligands for either molecules such as integrin receptors, molecules with high charge density such glycosaminoglycans (*e.g*., hyaluronan) or proteoglycans, or other adhesins). Structural functions retained by useful acellular matrices include maintenance of histological architecture, maintenance of the three-dimensional array of the tissue's components and physical characteristics such as strength, elasticity, and durability, defined porosity, and retention of macromolecules. The efficiency of the biological functions of an ATM can be measured, for example, by the ability of the ATM to support cell (*e.g*., epithelial cell) proliferation and is at least 50% (*e.g*., at least: 50%; 60%; 70%; 80%; 90%; 95%; 98%; 99%; 99.5%; 100%; or more than 100%) of that of the native tissue or organ from which the ATM is made.

It is not necessary that the ATM be made from tissue that is identical to the surrounding host tissue but should simply be amenable to being remodeled by invading or infiltrating cells such as differentiated cells of the relevant host tissue, stem cells such as mesenchymal stem cells, or progenitor cells. It is understood that the ATM can be produced from any collagen-containing soft tissue and muscular skeleton (*e.g*., dermis, fascia, pericardium, dura, umbilical cords, placentae, cardiac valves, ligaments, tendons, vascular tissue (arteries and veins such as saphenous veins), neural connective tissue, urinary bladder tissue, ureter tissue, or intestinal tissue), as long as the above-described properties are retained by the matrix.

An ATM useful for the invention can optionally be made from a recipient's own collagen-based tissue. Furthermore, while an ATM will generally have been made from one or more individuals of the same species as the recipient of the BGC, this is not necessarily the case. Thus, for example, an ATM can have been made from a porcine tissue and be used to make a BGC that can be implanted in a human patient. Species that can serve as recipients of a BGC and donors of tissues or organs for the production of the ATM component of the BGC can include, without limitation, humans, non-human primates (*e.g*., monkeys, baboons, or chimpanzees), pigs, cows, horses, goats, sheep, dogs, cats, rabbits, guinea pigs, gerbils, hamsters, rats, or mice.

Of particular interest as donors are animals (*e.g*., pigs) that have been genetically engineered to lack the terminal galactose-*α*-1, 3-galactose moiety. For descriptions of appropriate animals see co-pending U.S. Published Application No. 2005/0028228 A1 and U.S. Patent No. 6,166,288. A major problem of xenotransplantation in recipient animals (*e.g*.., humans) that do not express the enzyme UDP-galactose:β-D-galactosyl-1,4-N-acetyl-D-glucosaminide α-1,3 galactosyl-transferase (α-1,3 galactosyltransferase; "α-GT") that catalyzes the formation of the terminal disaccharide structure, galactose α-1,3 galactose ("α-gal"), is the hyperacute rejection of xenografts in such recipients. This rejection is largely, if not exclusively, due to the action of antibodies specific for the α-gal epitope on the surface of cells in the xenograft. Transgenic animals (*e.g*., pigs) have been derived which lack, or substantially lack, functional α-GT and thus also lack, or substantially lack, α-gal epitopes. Disclosed methods of making transgenic animals, and in particular gene-disrupted transgenic animals, are well known in the art. Methods of making gene-disrupted animals involve incorporating a disrupted form of a gene of interest into the germline of an individual of a species. The gene can be disrupted so that no protein product (*e.g*., α-GT) is produced or a protein product is produced that lacks the activity, or substantially lacks the activity, of the native protein. As used herein, a α-GT protein "substantially lacking α-GT activity" is an α-GT protein that has less than 5% (*e.g*., less than: 4%; 2%; 1%; 0.1%; 0.01%; 0.001%; or even less than 0.001%) of the ability of wild-type α-GT to generate α-gal epitopes. Disclosed methods of disrupting genes, and in particular, the α-GT gene, are known in the art and generally involve the process known as homologous recombination. In this process, one or both copies of a wild-type gene of interest can be disrupted by inserting a sequence into the wild-type gene(s) such that no transcript is produced from the gene(s); or a transcript is produced from which no protein is translated; or a transcript is produced that directs the synthesis of a protein that lacks, or substantially lacks, the functional activity of the protein of interest. Such constructs typically include all or part of the genomic sequence of the gene of interest and contain, within that genomic sequence, a sequence that will disrupt expression of the gene of interest in one of the ways described above. The sequence used to disrupt expression of the gene can be a sequence encoding a protein that confers antibiotic resistance (*e.g*., neomycin resistance) on target cells that have incorporated the construct into their genomes. Such a coding sequence facilitates the *in vitro* selection of cells that have incorporated the genetic construct into their genomes. Additional drug selection methodologies known in the art can be used to select cells in which recombination between the construct and at least one copy of the targeted gene has occurred.

In some disclosed methods of generating gene disrupted animals, totipotent cells (*i.e*., cells capable of giving rise to all cell types of an embryo) can be used as target cells. Such cells include, for example embryonic stem (ES) cells (in the form of ES cell lines) or fertilized eggs (oocytes). A population of ES cells in which at least one copy of the gene of interest is disrupted can be injected into appropriate blastocysts and the injected blastocysts can be implanted into foster mothers. Alternatively, fertilized eggs injected with the gene-disrupting construct of interest can be implanted in the foster mothers. Moreover, oocytes implanted in foster mothers can be those that have been enucleated and injected with, or fused with, nuclei from successfully gene-disrupted ES cells [Campbell et al., (1996) Nature 380: 64-66]. Resulting mutation-containing offspring arising in such mother foster mothers can be identified and, from these founder animals, distinct animal lines can be produced using breeding and selection methods known to those in the art. Disclosed embodiment: standard and gene-disrupted transgenic animals can also be produced using somatic cells (*e.g.,* fetal fibroblasts) as target cells for the gene-disruption. Such cells grow much faster and are more easily handled *in vitro* than, for example, ES cells, thus facilitating the gene disruption and subsequent gene-disrupted cell selection procedures. Once a line of gene-disrupted somatic cells has been selected *in vitro*, nuclei from the gene-disrupted somatic cells can be incorporated into totipotent cells (*e.g.,* ES cells or oocytes), which are then handled as described above. Methods for nuclear transplantation are known to those in the art and can include techniques such as, for example, cell fusion or nuclear transplantation. Disclosed embodiment: most commonly, the gene disruption procedures result in disruption of only one allele of a gene of interest. In these cases, the transgenic animals will be heterozygous for the disrupted gene. Breeding of such heterozygotes and appropriate selection procedures familiar to those in the art can then be used to derive animals that are homozygous for the disrupted gene. Naturally, such breeding procedures are not necessary where the gene disruption procedure described above resulted in disruption of both alleles of the gene of interest.

For the production of BGC, ATM in the form of fragments (*i.e*., particles, threads or fibers) are generally used (see below). The ATM can be produced by any of a variety of methods. All that is required is that the steps used in their production result in matrices with the above-described biological and structural properties. Particularly useful methods of production include those described in U.S. Patent Nos. 4,865,871; 5,366,616; 6,933,326 and copending U.S. Published Application Nos. 2003/0035843 A1, and 2005/0028228 A1. In brief, the steps involved in the production of an ATM generally include harvesting the tissue from a donor (*e.g*., a human cadaver or any of the above-listed mammals), chemical treatment so as to stabilize the tissue and avoid biochemical and structural degradation together with, or followed by, cell removal under conditions which similarly preserve biological and structural function. The ATM can optionally be treated with a cryopreservation agent and cryopreserved and, optionally, freeze-dried, again under conditions necessary to maintain the described biological and structural properties of the matrix. After freezing or freeze drying, the tissue can be fragmented, *e.g*., pulverized or micronized, to produce a particulate ATM under similar function-preserving conditions. All steps are generally carried out under aseptic, preferably sterile, conditions.

An exemplary method of producing ATM, which is described in greater detail in U.S. Patent No. 5,366,616, is summarized below.

After removal from the donor, the tissue is placed in an initial stabilizing solution. The initial stabilizing solution arrests and prevents osmotic, hypoxic, autolytic, and proteolytic degradation, protects against microbial contamination, and reduces mechanical damage that can occur with tissues that contain, for example, smooth muscle components (*e.g*., blood vessels). The stabilizing solution generally contains an appropriate buffer, one or more antioxidants, one or more oncotic agents, one or more antibiotics, one or more protease inhibitors, and in some cases, a smooth muscle relaxant.

The tissue is then placed in a processing solution to remove viable cells (*e.g*., epithelial cells, endothelial cells, smooth muscle cells, and fibroblasts) from the structural matrix without damaging the basement membrane complex or the biological and structural integrity of the collagen matrix. The processing solution generally contains an appropriate buffer, salt, an antibiotic, one or more detergents, one or more agents to prevent cross-linking, one or more protease inhibitors, and/or one or more enzymes. Treatment of the tissue must be (a) with a processing solution containing active agents at a concentration and (b) for a time period such that the structural integrity of the matrix is maintained.

After decellularization, the tissue can be frozen (*i.e*., cryopreserved) and optionally, freeze-dried. Before freezing, the tissue can be incubated in a cryopreservation solution. This solution generally contains one or more cryoprotectants to minimize ice crystal damage to the structural matrix that could occur during freezing. If the tissue is to be freeze-dried, the solution will generally also contain one or more dry-protective components, to minimize structural damage during drying and may include a combination of an organic solvent and water which undergoes neither expansion or contraction during freezing. The cryoprotective and dry-protective agents can be the same one or more substances. If the tissue is not going to be freeze-dried, it can be frozen by placing it (in a sterilized container) in a freezer at about -80°C, or by plunging it into sterile liquid nitrogen, and then storing at a temperature below -160°C until use. The sample can be thawed prior to use by, for example, immersing a sterile non-permeable vessel (see below) containing in a water bath at about 37°C or by allowing the tissue to come to room temperature under ambient conditions.

If the tissue is to be frozen and freeze-dried, following incubation in the cryopreservation solution, the tissue can be packaged inside a sterile vessel that is permeable to water vapor yet impermeable to bacteria, e.g., a water vapor permeable pouch or glass vial. One side of a preferred pouch consists of medical grade porous Tyvek® membrane, a trademarked product of DuPont Company of Wilmington, DE. This membrane is porous to water vapor and impervious to bacteria and dust. The Tyvek membrane is heat sealed to a impermeable polythylene laminate sheet, leaving one side open, thus forming a two-sided pouch. The open pouch is sterilized by irradiation (*e.g*., γ-irradiation) prior to use. The tissue is aseptically placed (through the open side) into the sterile pouch. The open side is then aseptically heat sealed to close the pouch. The packaged tissue is henceforth protected from microbial contamination throughout subsequent processing steps.

The vessel containing the tissue is cooled to a low temperature at a specified rate which is compatible with the specific cryoprotectant formulation to minimize the freezing damage. See U.S. Patent No. 5,336,616 for examples of appropriate cooling protocols. The tissue is then dried at a low temperature under vacuum conditions, such that water vapor is removed sequentially from each ice crystal phase.

At the completion of the drying of the samples in the water vapor permeable vessel, the vacuum of the freeze drying apparatus is reversed with a dry inert gas such as nitrogen, helium, or argon. While being maintained in the same gaseous environment, the semipermeable vessel is placed inside an impervious (*i.e*., impermeable to water vapor as well as microorganisms) vessel (*e.g*., a pouch) which is further sealed, e.g., by heat and/or pressure. Where the tissue sample was frozen and dried in a glass vial, the vial is sealed under vacuum with an appropriate inert stopper and the vacuum of the drying apparatus reversed with an inert gas prior to unloading. In either case, the final product is hermetically sealed in an inert gaseous atmosphere. The freeze-dried tissue may be stored under refrigerated conditions until fragmentation or, if desired, rehydration.

Particulate ATM have a generally spherical or even irregular shape, with the longest dimension being not greater than 1000 microns. Particulate ATM can be made from any of the above described non-particulate ATM by any process that results in the preservation of the biological and structural functions described above and, in particular, damage to collagen fibers, including sheared fiber ends, should be minimized.

One appropriate method for making particulate ATM is described in U.S. Patent No.6,933,326. The process is briefly described below with respect to a freeze-dried dermal ATM but one of skill in the art could readily adapt the method for use with frozen or freeze-dried ATM derived from any of the other tissues listed herein.

The acellular dermal matrix can be cut into strips (using, for example, a Zimmer mesher fitted with a non-interrupting "continuous" cutting wheel). The resulting long strips are then cut into lengths of about 1cm to about 2cm. A homogenizer and sterilized homogenizer probe (*e.g*., a LabTeck Macro homogenizer available from OMNI International, Warrenton, VA) is assembled and cooled to cryogenic temperatures (*i.e*., about -196°C to about -160°C) using sterile liquid nitrogen which is poured into the homogenizer tower. Once the homogenizer has reached a cryogenic temperature, cut pieces of ATM are added to the homogenizing tower containing the liquid nitrogen. The homogenizer is then activated so as to cryogenically fracture the pieces of ATM. The time and duration of the cryogenic fracturing step will depend upon the homogenizer utilized, the size of the homogenizing chamber, and the speed and time at which the homogenizer is operated, and are readily determinable by one skilled in the art. As an alternative, the cryofracturing process can be conducted in cryomill cooled to a cryogenic temperature.

The cryofractured particulate ATM is, optionally, sorted by particle size by washing the product of the homogenization with sterile liquid nitrogen through a series of metal screens that have also been cooled to a cryogenic temperature. It is generally useful to eliminate large undesired particles with a screen with a relatively large pore size before proceeding to one (or more screens) with a smaller pore size. Once isolated, the particles can be freeze-dried to ensure that any residual moisture that may have been absorbed during the procedure is removed. The final product is a powder (usually white or off-white) generally having a particle size of about 1 micron to about 900 microns, about 30 microns to about 750 microns, or about 150 to about 300 microns.

ATM fragments can also be fibers or threads. Such fibers or threads would generally not be greater than 5 cm (e.g., e.g., not greater than: 4.5 cm; 4.0 cm; 3.5 cm; 3.0 cm; 2.5 cm; 2.0 cm; 1.5 cm; 1.0 cm; 0.5 cm; 0.25 cm; 0.1 cm; 0.05 cm; or 0.02 cm) in length and not greater than 3 mm (e.g., not greater than: 2.5 mm; 2.0 mm; 1.5 mm; 1.0 mm; 0.5 mm; 0.2 mm; 0.1 mm; 0.05 mm; 0.02 mm; or 0.01 mm) at their widest point. Methods of producing fibers and threads from frozen or freeze-dried ATM would be apparent to those skilled in the art and include both manual or machine cutting of the frozen or freeze-dried ATM.

The fragmented ATM is readily rehydrated by suspension in normal saline or any other suitable rehydrating agent known in the art. It may also be suspended in any suitable carrier known in the art (see, for example, U.S. Patent No. 5,284,655 incorporated herein by reference in its entirety). If suspended at a high concentration (*e.g*., at about 600mg/mL), the fragmented ATM can form a "putty", and if suspended at a somewhat lower concentration (*e.g*., about 330 mg/mL), it can form a "paste". The above described method can be applied to any form of stored ATM, for example, fragmented ATM that has been freeze-dried or fragmented ATM that has been frozen.

One highly suitable freeze-dried ATM is produced from human dermis by the LifeCell Corporation (Branchburg, NJ) and marketed in the form of small sheets as AlloDerm®. Such sheets are marketed by the LifeCell Corporation as rectangular sheets with the dimensions of, for example, 1cm x 2cm, 3cm x 7cm, 4cm x 8cm, 5cm x 10cm, 4cm x 12cm, and 6cm x 12cm. The cryoprotectant used for freezing and drying AlloDerm® is a solution of 35% maltodextrin and 10mM ethylenediaminetetraacetate (EDTA). Thus, the final dried product contains about 60% by weight ATM and about 40% by weight maltodextrin. The LifeCell Corporation also makes an analogous product made from porcine dermis (designated XenoDerm™) having the same proportions of ATM and maltodextrin as AlloDerm®. In addition, the LifeCell Corporation markets a particulate acellular dermal matrix made by cryofracturing AlloDerm® (as described above) under the name Cymetra®. The particle size for Cymetra is in the range of about 60 microns to about 150 microns as determined by mass. The particles of particulate or pulverized (powdered) ATM will be less than 1.0 mm in their longest dimension. Pieces of ATM with dimensions greater than this are non-particulate acellular matrices.

### Demineralized bone matrix

As used herein, "demineralized bone matrix" (DBM) refers to bone that has been treated to remove all or substantially all, of the inorganic, mineral components. DBM from which substantially all the mineral component has been removed is bone matrix that has less than 5% of the mineral concentration of that found in the bone from which the DBM was prepared. The extraction procedures responsible for demineralization also remove all or substantially all viable cells from the bone matrix. DBM from which substantially all the viable cells have been removed is bone matrix that has less than 1% (*e.g*., less than: 0.1%; 0.01%; 0.001%; 0.0001%; 0.00001%; 0.000001%; or 0.0%) of the concentration of viable cells found in the bone from which the DBM was prepared.

The major protein component of DBM is collagen, which accounts for 70-90% of the non-mineralized component of bone matrix. Other non-collagenous matrix protein constituents include glycoproteins, *e.g*., osteonectin and thrombospondin; proteoglycans *e.g*., biglycan and decorin; sialoproteins, *e.g*., osteopontin and bone sialoprotein; and bone Gla proteins, *e.g*., osteocalcin. Bone matrix is rich in growth factors, a diverse group of peptides and small proteins that regulate the formation of new tissue through their effects on cell growth, function, and motility. DBM typically includes bone morphogenetic proteins (BMPs), a family of multi-functional growth factors with strong abilities to induce new bone or cartilage formation, as well as other growth factors such as transforming growth factor-*β*1 (TGF-*β*1) and insulin-like growth factor-1 (IGF-1) that play critical roles in the regulation of bone growth.

DBM possesses most, ideally all, the biological properties of native bone that are important for successful bone grafting. DBM is osteoinductive. The BMP's and other growth factors in DBM signal mesenchymal stem cells to differentiate into osteoprogenitor cells to produce new bone growth. Thus, DBM enables regeneration to occur throughout a defect in need of repair rather than just at the edges of a defect. DBM is also osteoconductive in that it supports neovascularization and invasion by osteoblasts.

DBM can be made from one or more individuals of the same species as the recipient of the BGC; it can also be made from individuals of a different species as the recipient of the BGC. Thus, for example, a DBM can have been made from a porcine tissue and be used to make a BGC that can be implanted in a human patient. Species that can serve as recipients of the BGC and donors of tissues or organs for the production of the DBM include, without limitation, humans, non-human primates (e.g., monkeys, baboons, or chimpanzees), pigs, cows, horses, goats, sheep, dogs, cats, rabbits, guinea pigs, gerbils, hamsters, rats, or mice. DBM can also be produced from animals (*e.g*., pigs) that have been genetically engineered to lack the terminal galactose-*α*-1, 3-galactose moiety. The production of such transgenic animals has been described above.

DBM can be produced from long bones, calvaria or any other type of bone. Typically, DBM can be derived, for example, from human cadaveric bone tissue that is harvested no longer than about 24 hours post mortem. Harvesting is done after reviewing the detailed medical history of the donor and the donor's family. The donor's blood is tested for markers for a variety of pathogenic conditions, e.g., hepatitis A antibody, hepatitis B surface antigen, hepatitis B core antibody, hepatitis C antibody, HIV-1 and -2 virus antibodies, HIV direct antigen, human T-cell lymphoma virus-1/2 antibody, syphilis, and aerobic and anaerobic bacteria. The surrounding tissues are also tested for surface and medullary contaminants.

DBM can be prepared by a variety of methods. All that is required is that the method used results in the production of DBM with the above described biological and structural properties (see above). Standards and guidelines for preparation of DBM from human tissues have been developed by the United States Pharmacopeia and the American Association of Tissue Banks. Essentially, bone samples are treated to remove tissue, blood and lipids, fragmented to a relatively uniform size, demineralized by extraction with acid, sterilized and freeze-dried for long term storage.

Following initial testing, bone samples are stripped of soft tissue. For transport, the bone can be incubated in a solution of antimicrobial agents, for example, bacitracin and polymixin B. The bone samples can then be cut into small pieces using methods known to those in the art, for example, using saws and grinders, and then treated to remove bone marrow, blood and lipids. Typical procedures for removal of bone marrow, blood and lipids can include incubation in either 70% ethanol or a 1:1 mixture of chloroform: methanol for 6 hours at room temperature. Demineralization can be carried out by extraction with acid, for example, 0.6 N hydrochloric acid for 3-24 hours. The acid extraction reduces the calcium content to less than 5% of that found in non-demineralized bone matrix. Methods of measuring bone calcium content are know to those in the art. The samples can then be rinsed with water and neutralized in a biologically compatible buffer, *e.g*., 0.1 M sodium phosphate. Other differential extraction procedures can also be included e.g., extraction with alkali-urea to remove molecules that may have inhibitory effects on osteoinduction [Behnam et al. (2004) Connective Tissue Research 45: 257-60]. DBM can also be prepared as described in U.S. Patent No. 5,284,655. For the production of BGC, DBM in the form of fragments (*e.g*., particles, fibers, and threads) are generally used (see below). The DBM fragments can be produced by any of a variety of methods. All that is required is that the steps used in their production result in matrices with the above-described biological and structural properties. Such procedures are similar to those used to make ATM fragments from frozen or dried (*e.g*., freeze-dried) ATM (see above). DBM fragments can be irregular in shape and have a non-uniform size distribution. Milling of the bone matrix into appropriately sized particles can be carried out at several points during preparation, *e.g*., following lipid removal or after acid extraction. The milling process is typically carried out using a standard grinder or blender and can be performed on either wet or dry bone matrix. DBM fibers can be produced as shavings from bone matrix. DBM particle sizes are generally the same as those for particulate ATM (see above). For example, the longest dimension for a DBM particle can range from 50-3000 microns; e.g., 100-250 microns; 100-500 microns; 100-850 microns; 100-1000 microns; 100-1500 microns; 100-2000 microns; 100-2500 microns; 100-3000 microns; 125-300 microns; 125-500 microns; 125-600 microns; 125-850 microns; 200-500 microns; 200-850 microns; 200-1000 microns; 200-1500 microns; 200-2000 microns; 200-3000 microns; 500-1000 microns; 500-1500 microns; 500-2000 microns; 500-2500 microns; 500-3000 microns; 1000-2000 microns; 1000-2500 microns; or 1000-3000 microns. Particles of particular sizes can be obtained using commercially available sieves. DBM fragments can be made as described in U.S. Patent Nos. 5,284,655 and 5,510,396. For long term storage, fragmented DBM can be subjected to one or more cycles of lyophilization. Lyophilization can be carried out as described for ATM (see above). Sterilization can be performed on the DBM samples using any standard method e.g., ethylene oxide treatment, γ-irradiation, electron-beam irradiation, x-ray irradiation, or ultra-violet irradiation.

DBM can also be obtained from commercial sources, including any AATT tissue bank, for example, LifeLink (Tampa, FL), LifeNet (Virginia Beach, VA) or AlloSource (Centennial, Colorado). Examples of DBM include Accell DBM100 (IsoTis OrthoBiologics, Irvine, California), Accell Connexus™ (IsoTis OrthoBiologics, Irvine, California), Allogro (AlloSource, Denver, Colorado), AlloMatrix Putty (Wright Medical Technologies, Arlington, Tennessee) DBX® Putty (Synthes, Paoli, Pennsylvania), DynaGraft™ (GenSci Regeneration Sciences and Innova Technologies Corporation, Toronto, Ontario, Canada), Grafton™ Allogenic Bone Matrix (Osteotech, Shrewsbury, New Jersey), InterGro™ Putty (Interpore Cross International, Irvine, California), Opteform (Exactech, Gainesville, Florida), Optium (LifeNet, Virginia Beach, Virginia), and Osteofil™ (Regeneration Technologies, Alachua, Florida).

One highly suitable preparation of particulate DBM is provided with AlloCraft-DBM™ (LifeCell Corporation, Branchburg, NJ).

### II. BGC preparation

### BGC formation

The BGC provided herein is a composite of fragmented ATM and fragmented DBM. The fragmented ATM and the fragmented DBM can be mixed in any ratio ranging, by weight, from 5% ATM: 95% DBM to 95% ATM: 5% DBM, *e.g*., 5% ATM: 95% DBM; 10% ATM: 90% DBM; 15% ATM: 85% DBM; 20% ATM: 80% DBM; 25% ATM: 75% DBM; 30% ATM: 70% DBM; 35% ATM: 65% DBM; 40% ATM: 60% DBM; 45% ATM: 55% DBM; 50% ATM: 50% DBM; 55% ATM: 45% DBM; 60% ATM: 40% DBM; 65% ATM: 35% DBM; 70% ATM: 30% DBM; 75% ATM: 25% DBM; 80% ATM: 20% DBM; 85% ATM: 15% DBM; 90% ATM: 10% DBM, 95% ATM: 5% DBM.

Any type of fragmented ATM and fragmented DBM can be combined. Thus, any form of fragmented ATM, for example, particles, threads or fibers can be combined with any form of fragmented DBM, for example, particles, threads or fibers. More than one type of fragmented ATM and fragmented DBM can also be combined e.g., the fragmented ATM can include a mixture of particles, fibers or threads of ATM and the DBM can include a mixture of particles, fibers or threads of DBM. Any combination of different forms of fragmented ATM and fragmented DBM can be used. Thus both the fragmented ATM and the fragmented DBM can have been freeze-dried; or either freeze-dried fragmented ATM or freeze-dried fragmented DBM can be combined with frozen fragmented ATM or frozen fragmented DBM, or both the fragmented ATM and the fragmented DBM can have been frozen.

The fragmented ATM / fragmented DBM mixtures used for BGC formation are substantially hydrated. Substantially hydrated mixtures can have moisture levels between 20% and 70%, *i.e*., at least 20-30% of fully hydrated matrices. Fully hydrated matrices are matrices that contain the maximum amount of bound and unbound water that it is possible for that matrix to contain under atmospheric pressure.

As used herein, the fragmented ATM / fragmented DBM mixtures which are substantially hydrated contain not less than 20% (*e.g*., not less than: 20%; 25%; 30%; 35%; 40%; 45%; 50%; 55%; 60%; 65%; 70%) of the water that the relevant fragmented ATM / fragmented DBM mixture contains when fully hydrated. As used herein, a "fully hydrated fragmented ATM / fragmented DBM mixture " is a fragmented ATM / fragmented DBM mixture containing the maximum amount of bound and unbound water that it is possible for that fragmented ATM / fragmented DBM mixture to contain under atmospheric pressure. In comparing the amounts of water (unbound and/or bound) in two (or more) fragmented ATM / fragmented DBM mixtures that are fully hydrated, since the maximum amount of water than a fragmented ATM / fragmented DBM mixture made from any particular tissue will vary with the temperature of the fragmented ATM / fragmented DBM mixture, it is of course important that measurements for the two (or more) fragmented ATM / fragmented DBM mixture be made at the same temperature. Bound water in a fragmented ATM / fragmented DBM mixture is the water in the fragmented ATM / fragmented DBM mixture whose molecular mobility (rotational and translational) is reduced (compared to pure bulky) due to molecular interactions (*e.g*., hydrogen bonding) between the water and the fragmented ATM / fragmented DBM mixture molecules and/or other phenomena (*e.g*., surface tension and geometric restriction) that limit the mobility of the water in the fragmented ATM / fragmented DBM mixture. Unbound water within the fragmented ATM / fragmented DBM mixture has the same molecular mobility properties as bulky water in dilute aqueous solutions such as, for example, biological fluids. As used herein, a "substantially hydrated fragmented ATM / fragmented DBM mixture" is a fragmented ATM / fragmented DBM mixture that contains, at atmospheric pressure, more than 20% (*e.g*., more than: 20%; 25%; 30%; 35%; 40%; 45%; 50%; 55%; 60%; 65%; 70%) of the unbound and/or bound water that the same fragmented ATM / fragmented DBM mixture would contain at atmospheric pressure when fully hydrated. Measurements of water amounts in the substantially hydrated and fully hydrated fragmented ATM / fragmented DBM mixture must be made at the same temperature.

As used herein, the term "ambient temperatures" means temperatures between 2°C to 30°C (e.g., 4°C to 10°C; 4°C to 15°C; 4°C to 25°C; 4°C to 30°C; 10°C to 15°C; 10°C to 20°C; 10°C to 25°C; 10°C to 30°C; 15°C to 20°C; 15°C to 25°C; 15°C to 30°C; 20°C to 25°C; 20°C to 25°C; 20°C to 30°C; or 25°C to 30°C).

With respect to freeze-dried fragmented ATM, it is important to minimize osmotic forces and surface tension effects during rehydration. The aim in rehydration is to augment the selective preservation of the extracellular support matrix. Appropriate rehydration can be accomplished by, for example, an initial incubation of the dried fragmented ATM in an environment of about 100% relative humidity, followed by immersion in a suitable rehydration solution. Alternatively, the dried fragmented ATM may be directly immersed in the rehydration solution, without prior incubation, in a high humidity environment. Rehydration should not cause osmotic damage to the sample. Vapor rehydration should ideally achieve a residual moisture level of at least 15% and fluid rehydration should result in a fragmented ATM moisture level of between 20% and 70%. Depending on the fragmented ATM to be rehydrated, the rehydration solution can be, for example, normal saline, PBS, Ringer's lactate, or a standard cell culture medium. Where the fragmented ATM is subject to the action of endogenous collagenases, elastases or residual autolytic activity from previously removed cells, additives to the rehydration solution are made and include protease inhibitors. Where residual free radical activity is present, agents to protect against free radicals are used including antioxidants, and enzymatic agents that protect against free radical damage. Antibiotics may also be included to inhibit bacterial contamination. Oncotic agents being in the form of proteoglycans, dextran and/or amino acids may also be included to prevent osmotic damage to the matrix during rehydration. Rehydration of a dry sample is especially suited to this process as it allows rapid and uniform distribution of the components of the rehydration solution. In addition, the rehydration solutions may contain specific components, for example, diphosphonates to inhibit alkaline phosphatase and prevent subsequent calcification. Agents may also be included in the rehydration solution to stimulate neovascularization and host cell infiltration following transplantation of the rehydrated extracellular matrix. The rehydration and the mixing steps can take place in any order. Thus, the fragmented ATM and the fragmented DBM can be rehydrated separately and then mixed or mixed and then rehydrated.

The fragmented ATM and the fragmented DBM can be mixed together in any method that maintains the osteoinductive properties of the BGC. For example, the fragmented ATM and the fragmented DBM can be placed in a bowl or container and mixed with a spatula. Another suitable method of mixing is via reciprocal motion of the fragmented ATM and fragmented DBM in two luer-locked syringes to form a homogeneous putty.

The consistency of the mixture used to make the BGC can vary depending upon the concentration of the matrix components. If the matrices are suspended at a relatively low concentration, the material can form a paste; at higher concentrations, the material can form a semi-solid putty. Those skilled in the art would be able, using entirely routine experimentation, to establish relative proportions of a fragmented ATM of interest and a fragmented DBM of interest to mix in order to obtain a mixture with a particular desired consistency.

Once the fragmented ATM and the fragmented DBM components have been mixed they can be molded into a shape suitable for use in bone grafting. The materials can be aliquotted directly into syringes for further processing. Alternatively, the mixture can be molded or shaped into any form that is a convenient size or shape for use in bone grafting. Such shapes can include, without limitation, sheets, cubes, rectangles, discs, wedges, spheres, ovals, cylinders, cones, or polyhedrons or any form that mimics the shape of native bone. The BGC can also be molded or shaped around a scaffold component prepared from any appropriate natural or synthetic material.

The mixture can be dried by any method known in the art that will result in the preservation of osteoinductivity, *e.g*., air drying, drying in atmosphere of, or under a stream of, inert gas (*e.g*., nitrogen or argon), or freeze-drying. Freeze-drying is a routine technique used in the art (see, for example, U.S. Patent Nos: 4,619,257; 4,676,070; 4,799,361; 4,865,871; 4,964,280; 5,024,838; 5,044,165; 5,154,007; 6,194,136; 5,336,616; 5,364,756; and 5,780,295) and suitable equipment is available from commercial sources such as Labconco (Kansas City, MI, USA). Freeze-drying involves the removal of water or other solvent from a frozen product by a process called sublimation. Sublimation occurs when a frozen liquid goes directly to the gaseous state without passing through the liquid phase. Those skilled in the art are well aware of the different freeze-drying methodologies available in the art [see, *e.g.,* "A Guide to Freeze-drying for the Laboratory"-an industry service publication by Labconco, (2004); and Franks (1994) Proc. Inst. Refrigeration. 91: 32-39]. Freeze-drying may be accomplished by any of a variety of methods, including, for example, the manifold, batch, or bulk methods.

Generally, the BGC is rehydrated prior to grafting or implantation. Alternatively, the BGC can be grafted or implanted without prior rehydration; in this case rehydration occurs *in vivo.* For rehydration, the BGC can be incubated in any biologically compatible solution, for example, normal saline, phosphate-buffered saline, Ringer's lactate or standard cell culture medium. The BGC is incubated in a solution for sufficient time for the BGC to become fully hydrated or to regain substantially the same amount of water as the mixture from which the BGC was made contains. Generally, the incubation time in the rehydration solution will be from about two minutes to about one hour, e.g., about five minutes to about 45 minutes, or about 10 minutes to about 30 minutes. The rehydration solution can optionally be replaced with fresh solution as many times as desired. This can be desirable where one or more of the water-replacing agents used in the water replacement process is not biologically compatible or is toxic. The temperature of the incubations will generally be ambient (*e.g*., room) temperature or can be at from about 15°C to about 40°C, *e.g*., at about 20°C to about 35°C, and the vessel containing the BGC and rehydration solution can be agitated gently during the incubation if so desired. Following rehydration, the BGC can be further shaped or molded into a form suitable for implantation.

The consistency the rehydrated BGC varies depending upon the amount of fluid added to the BGC. For example, if a relatively large amount of fluid is added, the BGC can form a paste upon its addition; if a relatively low amount of fluid is added, the BGC can form a semi-solid putty upon its addition. Those skilled in the art would be able, using entirely routine experimentation, to establish relative amounts of fluid to add to a BGC of interest in order to obtain a rehydrated BGC with a desired consistency.

The BGC can contain one or more (e.g., 2, 3, 4, 5, 6, 7, 8, 9, 10, 11, or 12) carrier substances (e.g., maltodextrin and polyhydroxyl compounds generally) that can enhance the ability of DBM to form paste-like or putty-like compositions upon rehydration. Generally the ATM components of the BGC provide this function and the BGC of the invention can contain no such carrier substances. Where the BGC do contain the carrier substances, they can be added to the mixture that is dried (e.g., by freeze-drying) to create the BGC (see above). Thus, for example, the ATM, the DBM, or both the ATM and DBM preparations used to create such mixtures, can contain the carrier substances. Alternatively, the carrier substances can be added to the BGC prior to, at the same time as, or after rehydration of the BGC. Suitable carrier substances, and in particular suitable polyhydroxyl compounds, are described in U.S. Patent Nos. 5,284,655 and 5,510,396, the disclosures of which are incorporated herein by reference in their entirety.

### Osteoinductivity

The BGC as provided herein is osteoinductive, *i.e*., it can induce new bone formation in or on bone tissue or in or on non-bone tissue in a recipient by stimulating the recruitment of bone-forming stem cells. While the invention is not limited by any particular mechanism of action, bone growth is generally mediated by three cell types, osteoblasts, osteocytes and osteoclasts which are responsible respectively for production, maintenance and resorption of bone. The osteoinductivity of the BGC can be evaluated by standard methods known to those in the art. Both in vivo and in vitro assays for osteoinductivity have been developed. In vivo models can include implanting BGC into an intramuscular site in an immune compromised animal model, *e.g.* a nude rat or mouse, or assaying the BGC in models of skeletal defects. The amount of bone produced at an implantation site can be evaluated by standard methods including histomorphometric analysis, measurements of calcium content, or enzymological assays that monitor levels of enzymes that are abundant in bone-forming osteoblasts, *e.g*., alkaline phosphatase. In vitro cell culture models can also be used to assess osteoinductive potential. Certain cell lines, *e.g*., Saos 2 osteosarcoma cells have been shown to proliferate when cultured in the presence of osteoinductive agents; an index of this proliferative activity can be correlated with the osteoinductive potential of the BGC.

### Storage

The dehydrated BGC can be stored for an extended period of time, *e.g*. 1 day, 2 days, 5 days, 1 week, 2 weeks, 1 month, 2 months, 3 months, 6 months, 9 months, 1 year, 2 years, 3 years. Storage can be at ambient temperature or under refrigeration, *e.g*., in liquid N₂ or at -80°C, -50°C, -20°C, -10°C, 0°C, 4°C, 10°C, 20°C, or 25°C.

Optionally, the BGC can be submitted to treatments to diminish the bioburden. This process is expected to decrease the level of infectious microorganisms within the BGC. As used herein, a process used to inactivate or kill "substantially all" microorganisms (*e.g*., bacteria, fungi (including yeasts), and/or viruses) in the BGC is a process that reduces the level of microorganisms in the BGC by least 10-fold (*e.g*., at least: 100-fold; 1,000-fold; 10⁴-fold; 10⁵-fold; 10⁶-fold; 10⁷-fold; 10⁸-fold; 10⁹-fold; or even 10¹⁰-fold) compared to the level in the BGC prior to the process. Any standard assay method may be used to determine if the process was successful. These assays can include techniques that directly measure microbial growth, *e.g*., the culture of swab samples on artificial growth media, or molecular detection methods, such as quantitative PCR. The BGC according to invention is irradiated, in particular the BGC can be exposed to γ-, x-, e-beam, and/or ultra-violet (wavelength of 10 nm to 320 nm, *e.g*., 50 nm to 320 nm, 100 nm to 320 nm, 150 nm to 320 nm, 180 nm to 320 nm, or 200 nm to 300 nm) radiation in order to decrease the level of, or eliminate, viable bacteria and/or fungi and/or infectious viruses. More important than the dose of radiation that the BGC is exposed to is the dose absorbed by the BGC. While for thicker BGC, the dose absorbed and the exposure dose will generally be close, in thinner BGC the dose of exposure may be higher than the dose absorbed. In addition, if a particular dose of radiation is administered at a low dose rate over a long period of time (*e.g*., two to 12 hours), more radiation is absorbed than if it is administered at a high dose rate over a short period of time (*e.g*., 2 seconds to 30 minutes). One of skill in the art will know how to test for whether, for a particular BGC, the dose absorbed is significantly less than the dose to which the BGC is exposed and how to account for such a discrepancy in selecting an exposure dose. Appropriate absorbed doses of γ-, x-, or e-beam irradiation can be 6 kGy - 45 kGy, *e.g*., 8 kGy - 38 kGy, 10 kGy - 36 kGy, 12 kGy - 34 kGy. Thus, the dose of γ-, x-, and or e-beam irradiation can be, for example, 12, 13, 14, 15, 16, 17, 18, 19, 20, 21,22, 23, 24, 25, 26, 27, 28, 29, 30, 31, 32, 33, or 34 kGy.

The BGC components, the fragmented ATM and the fragmented DBM, mixed or separated, can be irradiated (at any of the above doses) at any stage of the BGC preparation. In addition, the irradiation of the BGC can be the second or even third exposure of the components of the BGC to irradiation. Thus for example, the fragmented ATM and the fragmented DBM can be irradiated separately, mixed to form the BGC and then the BGC can be irradiated.

The BGC can also be sterilized using peracetic acid. (See U.S. Patent No. 5,460,962, the disclosure of which is incorporated herein by reference in its entirety.) Thus, the peracetic acid can be added, for example, to the fragmented ATM/ fragmented DBM mixture prior to the drying of the mixture.

Generally, the BGC is transported to the appropriate hospital or treatment facility prior to rehydration and the rehydration is performed by clinical personnel immediately prior to grafting or implanting. However, rehydration can be performed prior to transportation to the hospital or treatment facility; in this case the BGC will generally be transported under refrigerated conditions. Transportation may be accomplished via standard carriers and under standard conditions relative to normal temperature exposure and delivery times.

### III. Treating Bone Disorders

### Bone disorders

The BGC provided herein are useful for treating any of a wide range of bone disorders that require amelioration or repair. Bone disorders can arise from diverse medical conditions, for example, traumatic injuries, congenital malformations, oncologic resections, and infection. Common to all these disorders are defects in the healing site that are particularly vulnerable to delayed unions or non-unions. Thus, applications of bone grafting include augmenting fracture healing, reconstruction of skeletal defects, fusing joints, joint reconstruction procedures, comminuted calcaneal fractures, packing joints for arthrodesis, *i.e*., surgically induced or spontaneous fusion of a joint, filling gaps in bone that arise as the result of resection of bone tumors, and filling gaps in debrided infected bone.

Certain groups of patients are at high risk for bone disorders. The BGC provided herein can be used to treat conditions arising from these disorders. For example, patients with conditions that result in weak bones *e.g*., osteoporosis or osteogenesis imperfecta have a high propensity for fracture or severe injury. Other medical conditions can impede the normal process of fracture healing. Individuals at a disadvantage for acute bone healing include those with conditions in which bone resporbtion occurs at a higher rate than does bone deposition such as osteoporosis, osteopenia, Charcot's neuroarthropathy, as well as those patients exposed to agents that compromise bone healing e.g., chemotherapeutic agents, non-steroidal antiinflammatory drugs or systemic nicotine resulting from smoking.

Thus, the BGC provided herein can be used for the repair of bones with any of the above-described damage or defects. The BGC can be used in any of the forms and prepared by any of the processes listed above. Bones to which such methods of treatment can be applied include, without limitation, long bones (*e.g*., tibia, femur, humerus, radius, ulna, or fibula), bones of the hand and foot (*e.g*., calcaneas bone or scaphoid bone), bones of the head and neck (*e.g*., temporal bone, parietal bone, frontal bone, maxilla, mandible), or vertebrae. As mentioned above, critical gap defects of bone can be treated with the BGC. In such critical gap defects, the gaps can be filled with, for example, a putty of the rehydrated BGC or with any form of molded and shaped BGC as described above.

The BGC can also be used to aid in incorporation of other bone graft materials, scaffolds or supportive structural devices. For example, the BGC can be used in conjunction with a transplant or implant displaying mechanical strength, to augment cortical grafts, or in lengthening procedures to increase the connectivity of the structural graft with the host bone. These other bone graft materials, scaffolds or supportive structural devices can include metals, ceramics and natural and synthetic polymers. Ceramic materials can include ceramics derived from natural sources e.g., coralline hydroxyapatite or synthetic compounds *e.g*., synthetic hydroxyapatite or *β-*tricalcium phosphate. Natural polymers useful in bone grafting can include starch, fibrin, collagen, chitosan, hyaluronic acid and polyhydroxybutyrate. Suitable synthetic polymers can be poly(*α*-hydroxy acids, poly (*ε*-caprolactone, poly(propylene fumarates) poly (BPA iminocarbonates, poly (phosphazenes). It is understood that such additional scaffold or physical support components can be in any convenient size or shape, *e.g*., sheets, cubes, rectangles, discs, or spheres.

BGC can be: (a) wrapped around a bone that is damaged or that contains a defect; (b) placed on the surface of a bone that is damaged or has a defect; (c) rolled up and inserted into a cavity, gap, or space in the bone; or (d) placed at a non-bony site to induce bone formation. One or more (*e.g*., one, two, three, four, five, six, seven, eight, nine, ten, 12, 14, 16, 18, 20, 25, 30, or more) such BGC's can be used at any particular site. The grafts can be held in place by, for example, sutures, staples, tacks, or tissue glues or sealants known in the art. Alternatively, if, for example, packed sufficiently tightly into a defect or cavity, they may need no securing device.

It is understood that the BGC can be applied to a tissue or organ in order to repair or regenerate that bone and/or a neighboring bone. Thus, for example, a BGC can be inserted into a critical gap defect of a long bone to generate a perisoteum equivalent surrounding the gap defect and the periosteum equivalent can in turn stimulate the production of bone within the gap in the bone. Similarly, a BGC can be implanted in a dental extraction socket to promote regeneration of any bone in the base of the socket that may have been lost as a result, for example, of tooth extraction. A BGC can also be used in spinal fusion.

### Delivery of therapeutic agents

The BGC can also be used as a scaffold for formation of new bone and/or as a vehicle for delivery of agents that aid in bone healing and new bone formation. These agents can include cells, growth factors or small molecule therapeutics. These agents can be incorporated into the BGC prior to the matrices being placed in the subject. Alternatively, they can be injected into the BGC already in place in a subject. These agents can be administered singly or in combination. For example, a BGC can be used to deliver cells, growth factors and small molecule therapeutics concurrently, or to deliver cells plus growth factors, or cells plus small molecule therapeutics, or growth factors plus small molecule therapeutics.

Naturally, administration of the agents mentioned above can be single, or multiple (*e.g*., two, three, four, five, six, seven, eight, nine, 10, 15, 20, 25, 30, 35, 40, 50, 60, 80, 90, 100, or as many as needed). Where multiple, the administrations can be at time intervals readily determinable by one skilled in art. Doses of the various substances and factors will vary greatly according to the species, age, weight, size, and sex of the subject and are also readily determinable by a skilled artisan.

Histocompatible, viable cells can be restored to the BGC to produce a permanently accepted graft that may be remodeled by the host. Cells can be derived from the intended recipient or an allogeneic donor. Cell types with which the BGC can be repopulated include, but are not limited to, embryonic stem cells (ESC), adult or embryonic mesenchymal stem cells (MSC), prochondroblasts, chondroblasts, chondrocytes, pro-osteoblasts, osteocytes, osteoclasts, monocytes, hematopoetic stem cells, gingival epithelial cells, endothelial cells, fibroblasts, or periodontal ligament stem cells. Any combination of two or more of these cell types (*e.g*., two, three, four, five, six, seven, eight, nine, or ten) may be used to repopulate the BGC. Methods for isolating specific cell types are well-known in the art. Donor cells may be used directly after harvest or they can be cultured in vitro using standard tissue culture techniques. Donor cells can be infused or injected into the BGC *in situ* just prior to placing of the BGC in a mammalian subject. Donor cells can also be cocultured with the BGC using standard tissue culture methods known to those in the art.

Growth factors that can be incorporated into the BGC include any of a wide range of cell growth factors, angiogenic factors, differentiation factors, cytokines, hormones, and chemokines known in the art. Any combination of two or more of the factors can be administered to a subject by any of the means recited below. Examples of relevant factors include bone morphogenetic proteins (BMP's), in particular, BMP 2, 4, 6, and 7 (BMP-7 is also called OP-1), fibroblast growth factors (FGF) (*e.g*., FGF1-10), epidermal growth factor, keratinocyte growth factor, vascular endothelial cell growth factors (VEGF) (*e.g*., VEGF A, B, C, D, and E), platelet-derived growth factor (PDGF), insulin-like growth factor (IGF) I and IGF-II, interferons (IFN) (*e.g*., IFN-*α*, *β*, or *γ*), transforming growth factors (TGF) (*e.g*., TGF*α* or *β*), tumor necrosis factor-*α*, an interleukin (IL) (*e.g*., IL-1 - IL-18), Osterix, Hedgehogs (*e.g*., sonic or desert), SOX9, parathyroid hormone, calcitonin prostaglandins, or ascorbic acid.

Factors that are proteins can also be delivered to a recipient subject by administering to the subject: (a) expression vectors (*e.g*., plasmids or viral vectors) containing nucleic acid sequences encoding any one or more of the above factors that are proteins; or (b) cells that have been transfected or transduced (stably or transiently) with such expression vectors. Such transfected or transduced cells will preferably be derived from, or histocompatible with, the recipient. However, it is possible that only short exposure to the factor is required and thus histo-incompatible cells can also be used.

The BGC can also be used as a vehicle for localized small molecule drug delivery. Osteomyelitis, *i.e*., infection of bone, is generally treated by curettage of the infected region, followed by bone grafting and long-term systemic administration of antibiotics. Incorporation of antimicrobial agents into the BGC can provide local high concentrations of antibiotics, thus minimizing the risk of adverse effects associated with long term high systemic doses. An antimicrobial agent can be an antibiotic. Examples of antibiotics include, without limitation, any representative classes of antibiotics e.g., 1) the aminoglycosides, such as gentamycin, kanamycin, neomycin, streptomycin or tobramycin; 2) the cephalosporins, such as, cefaclor, cefadroxil or cefotaxime; 3) the macrolides, such as azithromycin, clarithromycin, or erythromycin; 4) the penicillins, such as amoxicillin, carbenicillin or penicillin; 5) the peptides, such as bacitracin, polymixin B or vancomycin; 6) the quinolones, such as ciprofloxacin, levofloxacin, or enoxacin; 7) the sulfonamides, such as sulfamethazole, sulfacetimide; or sulfamethoxazole; 8) the tetracyclines, such as doxycycline, minocycline or tetracycline; 8) other antibiotics with diverse mechanisms of action such as rifampin, chloramphenicol, or nitrofuratoin.

The BGC can also be used as a delivery vehicle for other antimicrobial agents, e.g., antifungal agents and antiviral agents.

The BGC can also be used for localized delivery of chemotherapeutic agents. Malignant bone tumors are typically treated by tumor resection and systemic administration of anticaneer drugs. Incorporation of anticancer agents into the BGC can provide local high concentrations of chemotherapy, thus mitigating the toxicity associated with long term high systemic doses. Examples of classes of chemotherapeutic agents include, without limitation, 1) alkylating agents *e.g*., cyclophosphamide; 2) anthracyclines *e*.*g*., daunorubicin, doxorubicin; 3) cycloskeletal disruptors *e.g*., paclitaxel; 4) topoisomerase inhibitors, *e.g*., etoposide; 5) nucleotide analogues *e.g*., azacitidine, fluorouracil, gemcitabine; 6) peptides *e.g*., bleomycin; 7) platinum-based agents *e.g*., carboplatin, cisplatin; 8) retinoids *e.g*., all-trans retinoic acid; and 9) vinca alkaloids *e.g*., vinblastine or vincristine.

### IV. Articles of Manufacture

The BGC provided herein can be included in an article of manufacture or as a kit. In one embodiment, the kit can include the BGC, packaging material, or a package insert, comprising instructions for a method of treatment. The packaging material can include components that promote the long term stability and sterility of the BGC. In another embodiment, a kit can include BGC components, so that the user can prepare the BGC directly. Such kits can include a fragmented DBM component, a fragmented ATM component, instructions for a method of mixing the components to make the BGC and suitable packaging materials. The kits can also include biologically compatible buffers for hydration of the components and the BGC.

A number of embodiments of the invention have been described. Nevertheless, it will be understood that various modifications may be made without departing from the scope of the invention. Accordingly, other embodiments are within the scope of the following claims.

### EXAMPLES

### Example 1

### Methods and Materials

### Preparation of demineralized bone matrix

The DBM used in the experiments described in Examples 4 and 5 was a component of the AlloCraft DBM™ kit made by LifeCell corporation and was supplied by LifeLink. DBM can be prepared as follows. Cadaveric bone samples are washed to remove soft tissue and blood. The washed bone is cut into small pieces, disinfected via a series of incubations in hydrogen peroxide and alcohol, and ground into particles of 125-850 microns in size.

Following treatment with dilute HCl to reduce calcium content to less than 5%, the resulting DBM is freeze-dried and used for the experiments described in Examples 4 and 5 below.

### Preparation of acellular tissue matrix

In the experiments described in Examples 4 and 5 below, the relevant ATM were produced using LifeCell's proprietary methodology and were components of the AlloCraft-DBM™ kits made by LifeCell Corporation. Where the ATM was made from dermis, it is referred to as acellular dermal matrix (ADM). Human donor skin was obtained from various U.S. tissue banks and hospitals throughout the U.S. that collected skin samples from deceased donors after obtaining consent from family members.

Procured skin was placed in RPMI 1640 tissue culture medium containing antibiotics (penicillin and streptomycin) and was shipped to LifeCell's facility in Branchburg, New Jersey, on wet ice, in the same medium. On arrival, the temperature of the skin tissue container was measured and the skin tissue was discarded if the temperature was above 10°C. The RPMI 1640 medium was changed under aseptic condition and the skin was stored at 4°C while serological tests for various pathogens (*Treponema pallidum* (tested for by the RPR and VDRL methods), HIV (human immunodeficiency virus) I and II, hepatitis B virus, hepatitis C virus, and HTLV (human T-lymphotropic virus) I and II) were performed on a sample of the skin. The skin was discarded if any of the pathogens were detected. Otherwise, it was transferred to a pre-freezing aqueous solution of 35% weight to volume (w/v) maltodextrin (M180) in phosphate buffered saline (PBS). After 2 to 4 hours at room temperature (20°C to 25°C), the solution containing the skin was frozen at -80°C and stored in a -80°C freezer until it was processed as described below.

Frozen skin was thawed at 37°C in a water bath until no ice was visible. The residual liquid was drained and the skin was submitted to the following processing steps: (i) de-epidermization; (ii) de-cellularization; (iii) wash.
(i) De-epidermization: Skin epidermis was removed by incubating the tissue sample with gentle agitation in a de-epidermizing solution (1 M NaCl, 0.5% w/v Triton X100, 10 mM ethylenediaminetetraacetic acid (EDTA)) for 8 - 32 hours at room temperature. For pig skin, this incubation was performed for 30-60 hours at room temperature. The epidermal layer was physically removed from dermis. The epidermis was discarded and the dermis was subjected to further processing as described below.
(ii) Decellularization: In order to kill cells and remove cellular components and debris, the dermis was rinsed for 5 to 60 minutes with a decellularizing solution (2% w/v sodium deoxycholate, 10 mM EDTA, 10 mM HEPES buffer, pH 7.8 - 8.2) and then incubated with gentle agitation in a fresh lot of the same solution for 12-30 hours at room temperature.
(iii) Wash: The washing regimen serves to wash out dead cells, cell debris, and residual chemicals used in the previous processing steps. The decellularized dermis was transferred to a first wash solution (phosphate buffered saline (PBS) containing 0.5% w/v Triton X-100 and 10 mM EDTA), which was then incubated with gentle agitation for 5 to 60 minutes at room temperature. The dermis was then subjected to three sequential washes in a second wash solution (PBS containing 10 mM EDTA) with gentle agitation at room temperature. The first two washes were short (15- 60 minutes each) and the third wash was long (6-30 hours).

After the wash regimen, the resulting acellular tissue matrix was used to prepare particulate ATM as described above in the "Acellular tissue matrices" section.

**Preparation of BGC.** BGC were prepared from two components, the particulate ATM and the particulate DBM, supplied with the AlloCraft-DBM™ kits. The particulate ATM included in the kit was Cymetra, an acellular dermal matrix provided as a micronized particulate (50-150 µm) form of AlloDerm®, that had been washed to remove the cryoprotectant and resuspended in sterile saline. To prepare the particulate ATM component, 5 g of Cymetra was suspended in about 200 mL of sterile 0.9% NaCl saline (Irrigation USP, Abbott, Laboratories, Abbott Park, Illinois) in a plastic cup, stirred and then centrifuged. The supernatant was removed and 10 mL of saline were added to the cup to resuspend the ATM. 8 mL of resuspended ATM were then transferred to a 10mL syringe. To prepare the particulate DBM, 1 mL (∼0.4g) of particulate DBM was placed in a 3 mL syringe and hydrated by the addition of 1 mL of sterile saline. The hydrated particulate ATM (8 mL) and hydrated particulate DBM (6 mL) were mixed by a syringe-to-syringe mixing via reciprocal motion of two luer-locked syringes. The mixture was then transferred in 1 mL aliquots into a series of 3 mL syringes, frozen at -80°C and then vacuum dried under standard conditions using a freeze-dryer (Savant Modulyo Freeze drying system, Newark, NJ). The syringes were then capped with sealing end caps and sealed in foil pouches. The resulting bone graft composition was cylindrical in shape and approximately 15 mm long and 0.7 mm in diameter. For use, the freeze-dried bone graft composition was hydrated with 1.0 mL of saline for 15 minutes prior to implantation.

**Sample irradiation.** Samples were γ-irradiated at -80°C in an insulated dry-ice container at STERIS Isomedix Services (Whippany, NJ) at a delivered dose of 26.6-28.9 kGy. Samples were e-beam irradiated at ambient temperature at Titan Scan Technologies (Denver, CO) at a delivered dose of 14-16 kGy.

**Animal preparation and sample implantation.** The animal implant studies were conducted at Toxikon Laboratories (Bedford, MA). Athymic nude rats were anesthetized and a pouch was surgically created in the hind leg between the semimembranous and adductor brevis muscles. Approximately 0.1-0.2 mL of test material was delivered via a syringe into the muscle pouch. After 28 days, the rats were euthanized with CO₂ gas and the implants were surgically removed. The biopsy sample from each site was divided into two equal parts. One half was fixed in 10% neutral buffered formalin and processed for histology. The other half was frozen at - 80°C and processed for analysis of alkaline phosphatase activity.

**Histological evaluation of new bone formation.** The biopsy samples were evaluated histologically for the presence of both newly deposited cartilage and newly mineralized bone matrix. The fixed samples were processed for histology using standard techniques and stained with hematoxylin/eosin and toluidine blue. Microscopic analysis was performed on coded samples. Each sample was scored for the presence of new bone formation on a scale of 0-5 based on the following scoring system:
0 = no new bone.
1 = at least 1 site of new bone formation.
2 = more than 1 site of new bone with <10% area of new bone in the total biopsy.
3 = multiple sites of new bone with >10% area of new bone in the total biopsy, though non-uniform distribution of the sites.
4 = multiple and significant number of sites of new bone well distributed with uniform distribution throughout the biopsy.
5 = extensive new bone formation throughout the entire biopsy representing a majority of the biopsy.

**Enzymological evaluation of new bone formation.** Osteoblasts, or bone forming cells, synthesize the enzyme alkaline phosphatase which plays a key role in the mineralization of new bone. Levels of alkaline phosphatase activity in a sample thus can be an indicator of new bone formation.

Frozen biopsy samples were thawed and dissected clean to be visually free of muscle tissue. The samples were homogenized in AP buffer (1.2 M 2-amino-2-methyl-1-propanol, pH 10.5 at 25°C) and the resulting lysates were centrifuged to pellet unsolubilized debris. To perform the assay, 200 µL of the supernatant was added to 250µL AP buffer that contained the alkaline phosphatase substrate (p-nitrophenol phosphate, 4 mg/mL) and incubated for 30 minutes at 37 °C. The production of the n-nitrophenol product was monitored by spectrophotometry at 415 nm. A separate calibration curve (derived using known amounts of n-nitrophenol) was used to determine the amount of product generated by the biopsy samples. Total protein content of the extracts was measured using a BCA protein assay kit according to the supplier's directions (Pierce Biotechnology, Inc., Rockford, Illinois). The alkaline phosphatase activity in the experimental samples was normalized for protein concentration and expressed as µm pNP/mg protein/hr.

**Acceptance criteria.** A histology score of ≥ 1 and AP activity of ≥ 1 µm/mg protein/min were considered to be evidence of new bone formation.

### Example 2. Osteoinductivity of BGC: γ-ray sterilization.

**Experimental Design.** The osteoinductivity of the BGC was compared with that of formulations in which the particulate DBM component had been subject to treatments (summarized in Table 1, below) designed to systematically explore the relationship between storage conditions and osteoinductivity. Relevant variables included storage temperature, γ-ray sterilization, and the timing of mixing the particulate DBM and the particulate ATM components relative to storage of the particulate DBM, *i.e*., whether the particulate DBM and the particulate ATM were combined before or after storage of the particulate DBM. All experimental samples were stored for 6 months before implantation. Sixteen animals were used in this study. Samples were implanted in test animals as described above in Example 1, "Animal Preparation and Sample Implantation". Each animal received one implant in each hind limb. To control for variability between individual animals, the test material in each Group (A through H) was divided into 4 aliquots and each aliquot was implanted in a separate site in a different animal. After 28 days, the samples were biopsied and the osteoinductive potential of the samples was evaluated as described in Example 1 under "Histological evaluation of new bone formation" and "Enzymological evaluation of new bone formation".

The experimental groups were designated A through H and were subject to the following conditions. In Groups F and G, the particulate DBM was combined with particulate ATM to form a BGC. The BGC was prepared according the method ("Preparation of BGC") described in Example 1. That is, the particulate DBM and the particulate ATM were hydrated, combined to form a mixture, freeze-dried, γ-irradiated, and then stored for 6 months prior to implantation. Thus, the particulate DBM and the particulate ATM in these samples were combined prior to storage. Group F was stored at -80°C and Group G was stored at room temperature.

In Groups A and B, the particulate DBM (in powder form) was stored as a γ-irradiated dry powder. At the end of the storage period, the particulate DBM was hydrated, combined with hydrated particulate ATM and implanted. Thus the particulate DBM and the particulate ATM in these samples were combined after storage. Group A was stored at -80°C and Group B was stored at room temperature.

In Groups C, D, and E, the particulate DBM was stored as a paste, *i.e.* after hydration. For these samples, 1 mL (∼0.4 g) of particulate DBM was combined with 1 mL of saline as described above under "Preparation of BGC". The particulate DBM paste in Samples D and E was γ-irradiated following hydration and the particulate DBM paste in Sample C was not γ-irradiated. All the samples in this group were then stored and, at the end of the storage period, were combined with hydrated particulate ATM and implanted. Thus the particulate DBM and the particulate ATM in these samples were combined after storage. Group D was stored at -80°C and Groups C and E were stored at room temperature.

In Group H, a control for factors related to storage and irradiation, nonirradiated particulate DBM powder was hydrated and mixed with hydrated particulate ATM immediately before implantation. Thus the particulate DBM and the particulate ATM in this group were combined with no intervening storage period.

**Table 1: Experimental design**

| **Group** | **DBM storage form** | **ATM and DBM combined (relative to storage period)** | **DBM storage temp.** | **γ-irradiation** |
|---|---|---|---|---|
| A | powder | after storage | -80°C | yes |
| B | powder | after storage | RT | yes |
| C | paste | after storage | RT | no |
| D | paste | after storage | -80°C | yes |
| E | paste | after storage | RT | yes |
| F | BGC (dry) | before storage | -80°C | yes |
| G | BGC (dry) | before storage | RT | yes |
| H | powder | not applicable | no storage | no |

**Results.** The histology scores for each implanted aliquot (the columns labeled 1, 2, 3, and 4) are shown in Table 2. Both BGC implants (Groups F and G) had histology scores of ≥ 1 and therefore showed histological evidence of new bone formation.

**Table 2: Histology scores**

| **Group** | **DBM storage form** | **ATM and DBM combined(relative to storage period)** | **DBM storage temp.** | **γ-irradiation** | **Aliquot number** | | | | **Average** |
|---|---|---|---|---|---|---|---|---|---|
| | | | | | **1** | **2** | **3** | **4** | |
| A | powder | after storage | -80°C | yes | 2.5 | 2.0 | 1.5 | 2.3 | 2.1 |
| B | powder | after storage | RT | yes | 2.5 | 2.3 | 2.3 | 2.3 | 2.4 |
| C | paste | after storage | RT | no | 0.5 | 0 | 0 | 0 | 0.1 |
| D | paste | after storage | -80°C | yes | 2.5 | 1.3 | 2.3 | 2.3 | 2.1 |
| E | paste | after storage | RT | yes | 2.0 | 0.5 | 1.0 | 0.5 | 1.0 |
| F | BGC (dry) | before storage | -80°C | yes | 2.0 | 2.5 | 1.0 | 2.5 | 2.0 |
| G | BGC (dry) | before storage | RT | yes | 2.0 | ** | 4.0 | ** | 3.0 |
| H | powder | not applicable | no storage | no | 1.0 | 2.0 | 3.0 | 2.0 | 2.0 |

| | | | | | | | | | |
|---|---|---|---|---|---|---|---|---|---|
| **The implants were missing in these sections, so that biopsy samples were not analyzed. | | | | | | | | | |

The alkaline phosphatase activity scores for each implanted aliquot (the columns labeled 1, 2, 3, and 4) are shown in Table 3. Overall, the BGC samples (Groups F and G) showed higher levels of AP activity than did the comparable samples made from stored particulate DBM paste (samples D and E).

**Table 3: Alkaline phosphatase activity (µmol/mg protein/hr)**

| **Group** | **DBM storage form** | **ATM and DBM combined (relative to storage period)** | **DBM storage temp.** | **γ-irradiation** | **Aliquot number** | | | | **Average** |
|---|---|---|---|---|---|---|---|---|---|
| | | | | | **1** | **2** | **3** | **4** | |
| A | powder | after storage | -80°C | yes | 1.63 | - | 0.97 | 2.02 | 1.54 |
| B | powder | after storage | RT | yes | 0.8 | 1.39 | 1.88 | 1.81 | 1.47 |
| C | paste | after storage | RT | no | 0.57 | 0.57 | 1.08 | 0.69 | 0.72 |
| D | paste | after storage | -80°C | yes | 0.77 | - | 0.95 | 1.48 | 1.07 |
| E | paste | after storage | RT | yes | 0.53 | 0.60 | 0.72 | 0.87 | 0.68 |
| F | BGC (dry) | before storage | -80°C | yes | 1.45 | 1.58 | 1.37 | 0.92 | 1.33 |
| G | BGC (dry) | before storage | RT | yes | 0.54 | 2.02 | 0.45 | ** | 1.00 |
| H | powder | not applicable | no storage | no | 0.73 | 1.79 | 2.36 | ** | 1.62 |

| | | | | | | | | | |
|---|---|---|---|---|---|---|---|---|---|
| [**The implants were missing in these sections, so that biopsy samples were not analyzed.] | | | | | | | | | |

The osteoinductivity (OI) profiles of the samples are summarized in Table 4. Samples were considered osteoinductive when both the AP activity was ≥ 1 and the histology score was ≥ 1. Both BGC samples (Groups F and G) were osteoinductive; moreover, the BGC stored at ambient temperature (Group G) retained osteoinductivity whereas samples in which the DBM paste had been stored at room temperature (Group E) did not.

**Table 4: Osteoinductivity of test samples**

| **Group** | **DBM storage form** | **ATM and DBM combined (relative to storage period)** | **DBM storage temp.** | **γ-irradiation** | **AP activity avg. (µmol/mg protein/hr)** | **Histology score (avg.)** | **Osteoinductivity** |
|---|---|---|---|---|---|---|---|
| A | powder | after storage | -80°C | yes | 1.54 | 2.1 | Yes |
| B | powder | after storage | RT | yes | 1.47 | 2.4 | Yes |
| C | paste | after storage | RT | no | 0.72 | 0.1 | No |
| D | paste | after storage | -80°C | yes | 1.07 | 2.1 | Yes |
| E | paste | after storage | RT | yes | 0.68 | 1.0 | No |
| F | BGC (dry) | before storage | -80°C | yes | 1.33 | 2.0 | Yes |
| G | BGC (dry) | before storage | RT | yes | 1.0 | 3.0 | Yes |
| H | powder | not applicable | no storage | no | 1.62 | 2.0 | Yes |

Additional control samples not shown in the above tables consisted of a putty made by mixing the ATM and DBM prior to 6 months of storage, γ-irradiation, and implantation as described above. These samples had an average histology score of 1.0 and average AP activity of 0.8 and thus did not meet the above recited criterion for osteoinductivity.

### Example 3. Osteoinductivity of BGC: electron beam sterilization

**Experimental Design.** The osteoinductivity of the BGC was compared with that of formulations in which the particulate DBM component had been subject to treatments (summarized in Table 5, below) designed to systematically explore the relationship between storage conditions and osteoinductivity. Relevant variables included storage temperate, electron-beam (e-beam) sterilization, and the timing of mixing the particulate DBM and the particulate ATM components relative to storage of the particulate DBM, *i.e*., whether the particulate DBM and the particulate ATM were combined before or after storage of the particulate DBM. All experimental samples were stored for 6 months. Seventeen animals were used in this study. Samples were implanted in test animals as described above in Example 1, "Animal Preparation and Sample Implantation". Each animal received two implants, one in each hind limb, of 0.2 mL each. To control for variability between individual animals, the test material in each Group (A through G) was divided into 5 aliquots and each aliquot was implanted in a separate site in a different animal. After 28 days, the samples were biopsied and the osteoinductive potential of the samples was evaluated as described in Example 1 under "Histological evaluation of new bone formation". Alkaline phosphatase activity was assayed according to the method provided in "Enzymological evaluation of new bone formation" except that the biopsy samples were homogenized in 1% triton-x-100 in phosphate-buffered saline (PBS).

The experimental groups were designated A through G and were subject to the following conditions. In Groups F and G, the particulate DBM was combined with particulate ATM to form a BGC. The BGC was prepared according the method ("Preparation of BGC") provided in Example 1. That is, the particulate DBM and the particulate ATM were hydrated, combined to form a mixture, freeze-dried, γ-irradiated, and then stored for 6 months prior to implantation. Thus, the particulate DBM and particulate ATM in these samples were combined prior to storage. Group E was stored at -80°C and Group F was stored at room temperature.

In Groups A and B, the particulate DBM (in powder form) was stored as an e-beam irradiated dry powder. At the end of the storage period, the particulate DBM was hydrated, combined with hydrated particulate ATM and implanted. Thus the particulate DBM and the particulate ATM in these samples were combined after storage. Group A was stored at -80°C and Group B was stored at room temperature.

In Groups C and D, the particulate DBM was stored as a paste, *i.e.* after hydration. (For these samples, one mL (∼0.4g) of particulate DBM was combined with 1 mL of saline as described above under "Preparation of BGC". Following hydration, the particulate DBM paste in these groups was e-beam irradiated, stored and at the end of the storage period, combined with hydrated particulate ATM and implanted. Thus the particulate DBM and the particulate ATM in these samples were combined after storage. Group C was stored at -80°C and Group D was stored at room temperature.

In Group G, a control for factors related to storage and irradiation, nonirradiated particulate DBM powder was hydrated and mixed with hydrated particulate ATM immediately before implantation. Thus, the particulate DBM and the particulate ATM in this group were combined with no intervening storage period.

**Table 5: Experimental design**

| **Group** | **DBM storage form** | **ATM and DBM combined (relative to storage period)** | **DBM storage temp.** | **e-beam irradiation** |
|---|---|---|---|---|
| A | powder | after storage | -80°C | yes |
| B | powder | after storage | RT | yes |
| C | paste | after storage | -80°C | yes |
| D | paste | after storage | RT | yes |
| E | BGC (dry) | before storage | -80°C | yes |
| F | BGC (dry) | before storage | RT | yes |
| G | powder | not applicable | no storage | no |

**Results.** The histology scores for each implanted aliquot (the columns labeled 1, 2, 3, 4, and 5) are shown in Table 6. Both BGC groups showed histological evidence of new bone formation.

**Table 6: Histology scores**

| **Group** | **DBM storage form** | **ATM and DBM combined (relative to storage period)** | **DBM storage temp.** | **e-beam irradiation** | **Aliquot number** | | | | | **average** |
|---|---|---|---|---|---|---|---|---|---|---|
| | | | | | **1** | **2** | **3** | **4** | **5** | |
| A | powder | after storage | -80°C | yes | 2.5 | 1.0 | 2.0 | 3.5 | 2.5 | 2.3 |
| B | powder | after storage | RT | yes | 3.3 | ** | 3.3 | 1.5 | 3.3 | 2.9 |
| C | paste | after storage | -80°C | yes | 0 | 3.5 | 2.5 | 2.5 | 2.3 | 2.2 |
| D | paste | after storage | RT | yes | 2.0 | 1.5 | 2.0 | 2.0 | 2.3 | 2.0 |
| E | dried BGC | before storage | -80°C | yes | 3.5 | 2.0 | 3.5 | 2.5 | 3.5 | 3.0 |
| F | dried BGC | before storage | RT | yes | 2.8 | 3.0 | 3.0 | 2.5 | 2.5 | 2.8 |
| G | powder | not applicable | no storage | no | 2.3 | 3.3 | 2.5 | 2.0 | ** | 2.5 |

| | | | | | | | | | | |
|---|---|---|---|---|---|---|---|---|---|---|
| **The implants were missing in these sections, so that biopsy samples were not analyzed. | | | | | | | | | | |

The corresponding alkaline phosphatase activities for each implanted aliquot (the columns labeled 1, 2, 3, 4, and 5) are shown in Table 7. A majority of the BGC samples showed enzymological evidence of new bone formation.

**Table 7: Alkaline Phosphatase Activity (µmol/mg protein/hr)**

| **Group** | **DBM storage form** | **ATM and DBM combined (relative to storage period)** | **DBM storage temp.** | **e-beam irradiation** | **Aliquot number** | | | | | **Average ± S.E.** |
|---|---|---|---|---|---|---|---|---|---|---|
| | | | | | **1** | **2** | **3** | **4** | **5** | |
| A | powder | after storage | -80°C | yes | 1.97 | 1.59 | 3.63 | 1.54 | 5.48 | 3.0 ± 0.8 |
| B | powder | after storage | RT | yes | 8.2 | 0.62 | 1.27 | 0.57 | 3.34 | 2.8 ± 1.6 |
| C | paste | after storage | -80°C | yes | 0.58 | 0.83 | 0.52 | 0.83 | 1.66 | 0.8 ± 0.2 |
| D | paste | after storage | RT | yes | 0.47 | 0.65 | 0.46 | 0.49 | 0.53 | 0.52 ± 0.04 |
| E | BGC (dry) | before storage | -80°C | yes | 4.69 | 3.20 | 2.12 | 3.85 | 0.53 | 2.9 ± 0.8 |
| F | BGC (dry) | before storage | RT | yes | 0.44 | 0.78 | 1.18 | 0.8 | 1.17 | 0.9 ± 0.2 |
| G | powder | not applicable | no storage | no | 1.62 | 2.06 | 0.72 | 0.40 | ** | 1.2 ± 0.4 |

| | | | | | | | | | | |
|---|---|---|---|---|---|---|---|---|---|---|
| **The implants were missing in these sections, so that biopsy samples were not analyzed. | | | | | | | | | | |

The osteoinductivity (OI) profiles of the samples are summarized in Table 8. In this study, a sample was considered osteoinductive if histology score was ≥ 1. The bone graft composition samples stored at -80°C (sample E) showed higher osteoinductive activity than did comparable samples made from hydrated, stored paste (sample C). A similar trend was observed with bone graft composition samples stored at ambient temperature.

**Table 8: Osteoinductivity of test samples**

| **Group** | **DBM storage form** | **ATM and DBM combined (relative to storage period)** | **DBM storage temp.** | **e-beam irradiation** | **histology score (avg.)** | **AP activity avg. (µmol/mg protein/hr)** |
|---|---|---|---|---|---|---|
| A | powder | after storage | -80°C | yes | 2.3 | 3.0 ± 0.8 |
| B | powder | after storage | RT | yes | 2.9 | 2.8 ± 1.6 |
| C | paste | after storage | -80°C | yes | 2.2 | 0.8 ± 0.2 |
| D | paste | after storage | RT | yes | 2.0 | 0.52 ± 0.04 |
| E | BGC (dry) | before storage | -80°C | yes | 3.0 | 2.9 ± 0.8 |
| F | BGC (dry) | before storage | RT | yes | 2.8 | 0.9 ± 0.2 |
| G | powder | not applicable | no storage | no | 2.5 | 1.2 ± 0.4 |

Additional control samples not shown in the above tables consisted of a putty made by mixing the ATM and DBM prior to 6 months or 1 year of storage, e-beam irradiation, and implantation as described above. The samples stored for 6 months had an average histology score of 1.9 and average AP activity of 0.5 and thus, while they did meet the above recited criterion for osteoinductivity, they had lower histology scores (and AP activity) than any of the other samples listed in Table 8. The samples stored for 1 year had an average histology score of 0 and average AP activity of 0.2 and thus they did not meet the above recited criterion for osteoinductivity.

## Claims

1. A method of making a bone graft composition (BGC); **characterised in that** said method comprises:
combining fragments of an acellular tissue matrix (ATM) with fragments of demineralized bone matrix (DBM) to create a mixture, wherein the fragments of ATM and the fragments of DBM in the mixture are substantially hydrated; drying and irradiating the mixture the mixture to provide a form that has been dried and irradiated;
wherein said form can be stored for six months and then rehydrated to provide an osteoinductive BCG.

2. The method of claim 1, wherein the ATM comprises dermis from which all, or substantially all, viable cells have been removed.

3. The method of claim 1, wherein the ATM comprises a tissue from which all, or substantially all, viable cells have been removed, wherein said tissue is selected from the group consisting of fascia, pericardial tissue, dura, umbilical cord tissue, placental tissue, cardiac valve tissue, ligament tissue, tendon tissue, arterial tissue, venous tissue, neural connective tissue, urinary bladder tissue, ureter tissue, and intestinal tissue.

4. The method of claim 1, wherein the ATM and/or DBM is made from human tissue.

5. The method of claim 1, wherein the ATM and/or DBM is made from non-human mammalian tissue.

6. The method of claim 5, wherein the non-human mammal is genetically engineered to lack expression of α-1,3- galactosyl residues.

7. The method of any preceding claim, wherein the BGC or mixture is irradiated such that it absorbs 6 kGy to 30 kGy of the radiation.

8. A BGC made by the method of any of claims 1 to 7.

9. A BGC as described in any preceding claim, for use in the treatment of the human or animal body by surgery or therapy.

10. A BGC as described in claim 9 for the use described therein; wherein said treatment is the treatment of bone.

11. A BGC as described in claim 9 or 10 for the use described therein; wherein the BGC is used in combination with a supportive structural device.

12. An article of manufacture comprising a BGC as described in any preceding claim.

13. An article of manufacture according to claim 12;
wherein the article comprises packaging material, or a package insert, comprising instructions for a method of treatment; the method comprising
(i) identifying a mammalian subject as having a recipient organ, or tissue, in need of amelioration or repair; and
(ii) placing the BGC in or on the organ or tissue.

## Patentansprüche

1. Verfahren zur Herstellung einer Knochentransplantatzusammensetzung (BGC), **dadurch gekennzeichnet, dass** das Verfahren umfasst:
Kombinieren von Fragmenten einer azellulären Gewebematrix (ATM) mit Fragmenten von demineralisierter Knochenmatrix (DBM), so dass ein Gemisch erzeugt wird, wobei die Fragmente von ATM und die Fragmente von DBM in dem Gemisch im Wesentlichen hydratisiert vorliegen; Trocknen und Bestrahlen des Gemischs, so dass eine Form bereitgestellt wird, die getrocknet und bestrahlt wurde;
wobei die Form sechs Monate lang aufbewahrt und dann rehydratisiert werden kann, so dass eine osteoinduktive BCG erhalten wird.

2. Verfahren nach Anspruch 1, wobei die ATM Dermis umfasst, aus der alle oder im Wesentlichen alle lebensfähigen Zellen entfernt worden sind.

3. Verfahren nach Anspruch 1, wobei die ATM ein Gewebe umfasst, aus dem alle oder im Wesentlichen alle lebensfähigen Zellen entfernt worden sind, wobei das Gewebe aus der Gruppe ausgewählt ist, die aus Faszien, Perikardgewebe, Dura, Nabelschnurgewebe, Plazentagewebe, Herzklappengewebe, Bandgewebe, Sehnengewebe, Arteriengewebe, Venengewebe, neuralem Bindegewebe, Harnblasengewebe, Harnleitergewebe und Darmgewebe besteht.

4. Verfahren nach Anspruch 1, wobei die ATM und/oder DBM aus menschlichem Gewebe hergestellt ist.

5. Verfahren nach Anspruch 1, wobei die ATM und/oder DBM aus nicht-menschlichem Säugetiergewebe hergestellt ist.

6. Verfahren nach Anspruch 5, wobei das nichtmenschliche Säugetier derart gentechnisch verändert wurde, dass es keine α-1,3-Galactosylreste exprimiert.

7. Verfahren nach einem vorhergehenden Anspruch, wobei die BGC oder das Gemisch so bestrahlt werden, dass sie bzw. es 6 kGy bis 30 kGy der Strahlung absorbiert.

8. BGC, die durch das Verfahren nach einem der Ansprüche 1 bis 7 hergestellt worden ist.

9. BGC nach einem vorhergehenden Anspruch zur Verwendung bei der Behandlung des Körpers eines Menschen oder Tiers durch Operation oder Therapie.

10. BGC nach Anspruch 9 zur hier beschriebenen Verwendung, wobei es sich bei der Behandlung um die Behandlung von Knochen handelt.

11. BGC nach Anspruch 9 oder 10 zur hier beschriebenen Verwendung, wobei die BGC in Kombination mit einer unterstützenden Strukturvorrichtung verwendet wird.

12. Herstellungsgegenstand, umfassend eine BGC nach einem vorhergehenden Anspruch.

13. Herstellungsgegenstand nach Anspruch 12,
wobei der Gegenstand Verpackungsmaterial oder eine Packungsbeilage umfasst, die Anweisungen für ein Behandlungsverfahren umfasst, wobei das Verfahren umfasst
i) Identifizieren eines Säugetierindividuums, das ein Empfängerorgan oder ein -Gewebe aufweist, das eine Besserung oder Reparatur erfordert; und
ii) Platzieren der BGC in oder auf dem Organ oder Gewebe.

## Revendications

1. Procédé de fabrication d'une composition de greffe osseuse (CGO) ; **caractérisé en ce que** ledit procédé comprend :
la combinaison de fragments d'une matrice tissulaire acellulaire (MTA) avec des fragments de matrice osseuse déminéralisée (MOD) pour créer un mélange, où les fragments de MTA et les fragments de MOD du mélange sont substantiellement hydratés ; le séchage et l'irradiation du mélange pour obtenir une forme qui a été séchée et irradiée ;
où ladite forme peut être stockée pendant six mois avant d'être réhydratée pour obtenir une CGO ostéo-inductrice.

2. Procédé selon la revendication 1, où la MTA comprend du derme dont toutes, ou substantiellement toutes, les cellules viables ont été éliminées.

3. Procédé selon la revendication 1, où la MTA comprend un tissu dont toutes, ou substantiellement toutes, les cellules viables ont été éliminées, où ledit tissu est choisi dans le groupe constitué par les suivants : faisceaux, tissu péricardique, dure-mère, tissu du cordon ombilical, tissu placentaire, tissu de valvule cardiaque, tissu de ligament, tissu de tendon, tissu artériel, tissu veineux, tissu conjonctif neural, tissu de la vessie urinaire, tissu de l'urètre, et tissue intestinal.

4. Procédé selon la revendication 1, où la MTA et/ou la MOD est fabriquée à partir de tissus humains.

5. Procédé selon la revendication 1, où la MTA et/ou la MOD est fabriquée à partir de tissus mammaliens non humains.

6. Procédé selon la revendication 5, où le mammifère non humain est génétiquement conçu pour être exempt de l'expression de résidus α-1,3-galactosyle.

7. Procédé selon l'une quelconque des revendications précédentes, où la CGO ou le mélange sont irradiés de sorte à absorber 6 kGy à 30 kGy des rayonnements.

8. CGO fabriquée par le procédé selon l'une quelconque des revendications 1 à 7.

9. CGO selon l'une quelconque des revendications précédentes, pour utilisation dans le traitement de l'organisme humain ou animal par chirurgie ou thérapie.

10. CGO selon la revendication 9 pour l'utilisation décrite dans la présente invention ; où ledit traitement est le traitement de l'os.

11. CGO selon la revendication 9 ou 10 pour l'utilisation décrite dans la présente invention ; où la CGO est utilisée en combinaison avec un dispositif de structures de soutien.

12. Article manufacturé comprenant une CGO selon l'une quelconque des revendications précédentes.

13. Article manufacturé selon la revendication 12 ; où l'article comprend un matériel d'emballage, ou une notice d'emballage, comprenant des instructions relatives à une méthode de traitement ; la méthode comprenant
(i) l'identification d'un sujet mammifère comme ayant un organe ou tissu bénéficiaire nécessitant une amélioration ou une réparation ; et
(ii) le placement de la CGO dans ou sur l'organe ou le tissu.
